# EUROPEAN PATENT SPECIFICATION

(11) **EP 2 943 501 B1**
(45) Date of publication and mention of the grant of the patent: **26.06.2019**
(21) Application number: 13734148.3
(22) Date of filing: 30.05.2013
(51) Int. Cl.: C07K 1/22, B01D 15/38, B01J 20/286, B01J 20/32

(54) **COMPOSITIONS FOR BINDING AND SEPARATING OF PROTEINS**
ZUSAMMENSETZUNGEN ZUR BINDUNG UND TRENNUNG VON PROTEINEN
COMPOSITIONS DE LIAISON OU DE SÉPARATION DE PROTÉINES

(30) Priority: 30.05.2012 HU P1200327
(43) Date of publication of application: 18.11.2015
(73) Proprietor: BUDAPESTI MÜSZAKI ES GAZDASAGTUDOMANYI EGYETEM, 1111 Budapest (HU); Fermentia Mikrobiológiai Kft., 1045 Budapest (HU)
(72) Inventor: POPPE, László, 1173 Budapest (HU); BOROS, Zoltán, 1121 Budapest (HU); VÉRTESSY, Beáta, 1118 Budapest (HU); KOVÁCS, Klaudia, 5700 Budapest (HU); TÓTH, Alexandra, 3521 Miskolc (HU); HORNYÁNSZKY, Gabor, 1173 Budapest (HU); NAGY, József, 1112 Budapest (HU); ERDÉLYI, Balázs, 1045 Budapest (HU); ERDÉLYINÉ, Bódai, Viktória, 1135 Budapest (HU); SÁTORHELYI, Péter, 1118 Budapest (HU)
(74) Representative: Germus, Gábor
(86) International application number: PCT/HU2013/000050
(87) International publication number: WO 2013/179072

(56) References cited:
- WO-A2-2009/019012
- BIRGER ANSPACH ET AL: "Silica-based metal chelate affinity sorbents I. Preparation and characterization of iminodiacetic acid affinity sorbents prepared via different immobilization techniques", JOURNAL OF CHROMATOGRAPHY, ELSEVIER SCIENCE PUBLISHERS B.V, NL, vol. 672, no. 1-2, 24 June 1994 (1994-06-24), pages 35-49, XP026604605, ISSN: 0021-9673, DOI: 10.1016/0021-9673(94)80592-X [retrieved on 1994-06-24] cited in the application

## Description

### SUBJECT OF THE INVENTION

The subject of present invention is a composition for binding and separating proteins characterized by the general formula (II) 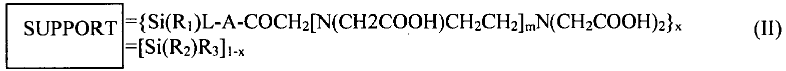 wherein
**R₁** and **R₂** are C₁-C₈ straight chain or branched alkyl, or C₂-C₈straight chain or branched alkenyl or alkynyl, C₆-C₈aryl or aralkyl or cycloalkyl group; or alkoxy group,
**R₃** is R₂ or a non-reactive group which contains straight chain, branched or cyclic structures formed by 2 - 30 covalently connected carbon, oxygen, sulfur, fluorine, chlorine, bromine or iodine atoms, **symbol** = represents one, two or three covalent single bonds between the Si atoms and the surface of the support, in case of one covalent bond, two of the R₁ - R₂ groups are attached to the given Si atom; in case of two covalent bonds, one of the R₁ - R₂ groups is attached to the given Si atom; in case of three covalent bonds, none of the groups R₁ - R₂ are attached to the given Si atom,
**A** is an atom or group of atoms containing a non-bonding pair of electrons, preferably NH, NR₂, S, O,
**L** is a straight, branched or cyclic structure connecting Si atoms and the A moiety comprising 2 - 22 carbon, sulfur or nitrogen atoms covalently connected to each other,
**x** is a number and 0 < x < 1,
**m** is an integer and m = 0, 1 or 2

Complexing the composition of general formula (II) with the appropriate lanthanide metal ion La, Ce, Nd, Pm, Sm, Eu, Gd, Dy, Ho, Er, Tm, Yb, Lu results in metal affinity carrier to which proteins and polypeptides tagged with oligohistidine affinity sequences can be bound and purified.

### BACKGROUND OF THE INVENTION

Proteins are of prime interest in modem biotechnology. Elucidation of genomes accelerated the cloning of genes and the elucidation of the structure and function of proteins. Significant development of the methods for the preparation of recombinant proteins facilitated their production, however, their structure and functions can only be studied in their pure state. Either the "classical" approach (isolation and purification of a protein of a given function) or the "neoclassical" one (preparation of a protein of given structure and investigation its role and structure thereafter) requires the elaboration of efficient methods of protein purification procedures.

For the implementation of protein purification both at the laboratory and industrial scale several methods are available [Burgess, R.R; Deutsche, M.P: Guide to Protein Purification (2nd Ed.), Methods in Enzymology, 2009; 463, Academic Press], among them a generally applied one is chromatography. Chromatography can be utilized advantageously in various domains of biotechnology, thus also for the purification of proteins, since with its aid precise separation of complex mixtures can be realized under mild conditions.

For the separation and purification of proteins among several other chromatographic techniques such as ion exchange chromatography (IEX), hydrophobic interaction chromatography (HIC), it is metal chelate affinity chromatography (MCAC) or under another name immobilized metal ion adsorption chromatography (IMAC) which is an especially suitable, sensitive and efficient method for the separation of most of the polypeptides and proteins [Block, H.; et al. Methods in Enzymology, 2009; 463:439-473]. Owing to chelate formation metal ions are strongly bound to the matrix, accordingly the adsorbed protein or polypeptide can be eluted by lowering the pH or competitive elution. In general, IMAC is used to separate proteins or molecules showing certain affinity to the transition metals in the matrix. Proteins can associate to the matrix through histidine, cysteine or tryptophan residing on their surface and having affinity to metal chelates. In case of some proteins the mode of bounding is not exactly known and can be influenced by multiple factors, such as e.g. the number, nature, relative position and accessibility to metal ions of the side chains on the surface capable of coordination. Therefore, it is difficult to predict which protein is able to bind and how tightly to the matrix.

Simple chelating agents have been used as ligands in IMAC, for instance iminodiacetic acid (IDA). Iminodiacetic acid bound to agarose and chelated with a variety of metals (Cu²⁺, Zn²⁺, Ni²⁺) and used to the immobilization of proteins and peptides is available commercially. Metal chelating resins have been used for the purification of interferon [US Patent 4,551,271], which also contained IDA ligands. The resin can be described by the following formula: [agarose]-O-CH₂-CH(OH)-CH₂-N(CH₂COO-)₂M²⁺, where M is nickel or copper. In the course of protein separations based on the IMAC principle the application of agarose based supports containing as chelator nitrilotriacetic acid (NTA) [Hochuli, E.; et al. Biotechnology, 1988;6:1321-1325 and US Patent 4,877,830] and the application as metal ions Ni²⁺ or Co²⁺ is most frequent where the matrix can be more precisely described by the following general formula:

[support]-spacer-NH-(CH₂)ₓCH(COOH)-N(CH₂COO)₂Ni²⁺ (x=2 or4)

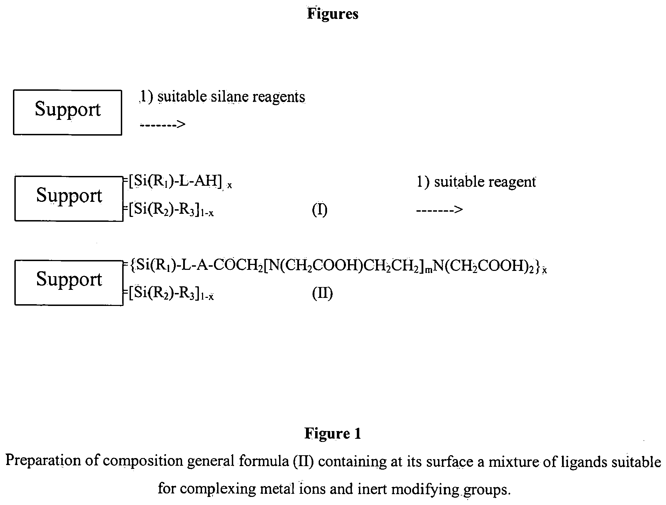

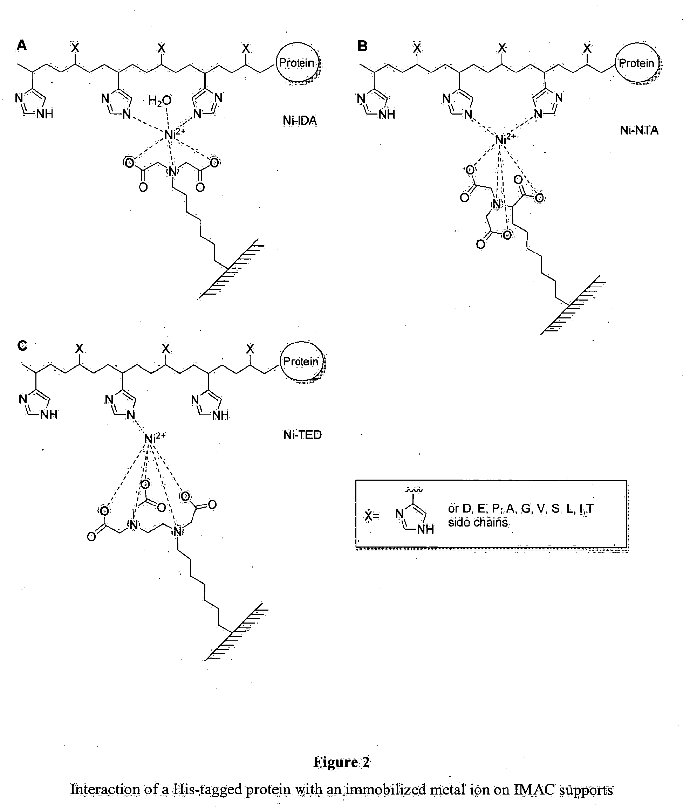

For protein separations based on the IMAC principle tris(carboxymethyl)ethylenediamine also proved to be operative [Porath, J.; Olin, B. Biochemistry, 1983;22:1621-1630]. More recent procedures describe also the preparation IMAC supports containing more complex tri- and tetradentate ligands or even their mixtures US Patent 6,623,655; US Patent 7,005,071; US Patent 7,709,612. The principle of IMAC based protein binding, that is the interaction between the His-tagged protein and the immobilized metal ion is depicted in Figure 2.

With the aid of the advanced methods of molecular biology proteins can nowadays be easily provided with fusion affinity segments containing one or more histidines (oligohistidine signal, His-tag) increasing thereby their affinity to the immobilized ligands containing metal ions.

As a consequence of this IMAC is playing an ever increasing role in the purification of proteins. In our days the most widely applied method for the purification of proteins based on the IMAC principle has become the purification of recombinant proteins genetically modified on the DNS template level extended by an oligohistidine sequence fusion marker [US Patent 4,569,794], According to a possible model, the binding of fusion oligohistidine sequences on IMAC supports containing immobilized metal ions is based on the interaction of the metal ion with one of the histidine side chains of the His-tag and with another to be found at a distance of two amino acids from the former (n - n+2). This is supported by the fact that metal ions immobilized on IMAC supports such His-tag sequences which contain histidine either in a consecutive or an alternating mode can also be seen in some examples shown in Table 1. Since consecutive His-tags are unstructured and therefore flexible, other patterns can also be envisaged even in a single molecule, such as e.g. n - n+1, n - +3, n - n+4, etc.

Examples for more complex oligohistidine tags are in US Patent 5,594,115 disclosing a fusion protein which can be characterized by the formula R₁-(His-X)ₙ-R₂ where (His-X)ₙ is a metal ion chelating peptide (n=3 - 6, where any of X can be Asp, Pro, Glu, Ala, Gly, Val, Ser, Leu, Ile or Thr), R₂ the target polypeptide, R₁ hydrogen or additional amino acid units.

**Table 1**

| Known His tag sequences (with single letter amino acid codes) | | |
|---|---|---|
| Sequence | His tag | Reference / Vector |
| H H H H H H | H₆ | US Patent 4,551,271 |
| H H H H H H H H | H₈ | pQE-TriSystem |
| H H H H H H H H H H | H₁₀ | pQE-TriSystem-5, -6 |
| | | |
| H Q H Q H Q H Q H Q H Q | (H Q)₆ | Prot. Expr. Purif. 1999; 15:389-400 |
| H N H N H N H N H N H N | (H N)₆ | US Patent. 7,176,298 |
| H G H G H G H G H G H Q | (H G/Q)₆ | Prot. Expr. Purif. 1999; 15:389-400 |
| H H Q H H A H H G | (HHX)₃ | Prot. Expr. Purif 1999; 15:389^400 |
| K D H L I H N V H K E H A H A H N K | HAT | US Patent.7,176,298 |
| (HX)₃₋₆^{a} | (HX)ₙ | US Patent: 5,594,115 |
| H X₁ H R H X₂ H ^{b} | (HXH)₂R | US Patent Appl..2004/0029781 |

| | | |
|---|---|---|
| ^{a}= X can be D, E, P, A, G, V, S, L, I and T; ^{b}= X\| can be A, R, N, D, Q, E, I, L, F, P, S, T, W, V; X₂ can be A, R, N, D, C, Q, E, G, I, L, K, M, P, S, T, Y, V | | |

Oligohistidine marking (His-tag) possessing chelating properties enables the purification of fusion proteins US Patent 4,569,794. The protein marked in this way can be separated from impurities in a way that it is passed through a column containing a metal immobilized onto an IMAC support. The His-tag of the fusion protein is bound to the matrix by chelation with the immobilized metal ion, while impurities having no affinity to the metal ions are eluted from the column. Thereafter by changing the conditions, typically by lowering the pH, with competing ligands (e.g. imidazole) or competing chelators (e.g.: EDTA) the His-tagged fusion protein can be eluted from the column and can be recovered from the eluate in a pure state.

In IMAC applications chelators binding various metal ions are typically bound to an agarose support [US Patent 4,551,271; US Patent 6,623,655; US Patent 7,005,071; US Patent 7,709,612. The advantage of natural polysaccharide based supports is that by functionalization with appropriate chelators they provide characteristically high capacity IMAC supports, while their disadvantage is that they can only be stored under special conditions (below room temperature, in a liquid state, protected from drying out), their recovery can only be realized in a limited way only for a couple of cycles, as well as their resistance to pressure and mechanical properties are also limited. As a consequence, up to the present day there is a demand for the development of both supports and chelators immobilizing the metal ions for the purpose of IMAC applications.

For chromatographic separations silica gel based supports can be applied advantageously [Encyclopedia of Chromatography (3rd Ed.), CRC Press, 2009]. Silica gel based phases modified on their surface have the advantageous property that they are mechanically sturdy and permit highly efficient separations [Majors, R E. LCGC, 1997; 15(11): 1008-1015]. Their additional advantageous feature is that in a dry state they can be stored for a long time, they do not swell on contact with. solvents and they can be applied at high pressures without deformation. Owing to these advantageous features IMAC supports based on silica gel have also been prepared. A typical example is when iminodiacetic acid (EDA) was reacted with glycidyloxypropyl-trimethoxysilane and the surface of silica gel was modified with the product obtained in this way [Anspach, F. B. Journal of Chromatography A, 1994; 672 (1-2):35-49], According to another method IMAC supports were prepared by modifying the surface of porous silica gel and other inorganic supports (e.g. porous glass, aluminum oxide, titanium-dioxide) with ethylenediamine tetraacetic acid (EDTA) and diethylenetriamine-pentaacetic acid (DTA) containing silanes [US Patent 4,835,269],

Silica gel based IMAC supports modified by silanes containing nitrilotriacetic acid (NTA) chelator complexed with metal ions proved to be suitable for binding and separation of His-tagged proteins [US Patent 7,112,552; US Patent 7,304,015; US Patent 7,304,016; US Patent 7,354,750 and WO2004/035170]. It is important to mention that the above approaches modified with the chelator containing moieties merely the surface of the inorganic support, typically the surface of porous silica gel.

It has to be emphasized that - as clearly shown by the examples in Table 1 - the chelating peptide generally contains alternating His units to be found at a distance of two amino acids (n - n+2). Consequently, if the chelator units are situated at the surface of the support in a controlled manner, at a given distance from each other, then multiple chelation and a more stable and/or more specific binding of the His-tagged protein comes into being. In a procedure involving the modification of the surface of mesoporous silica gel it is modified by a mixture composed of a varying ratio of para-aminophenyl-trimethoxysilane and phenyl-trimethoxysilane followed by coupling the amino groups with fluorescent groups, demonstrated that by varying the ratio of ligand-binding and non-binding silanes the surface of the silica gel can be modified by ligands having a controlled density and distance [Banet, P; et al. Langmuir, 2008; 24(16):9030-9037]. WO2009/019012 discloses EDTA-amide silica with a passivated surface and different ligand coupling densities.

At the same time, it is also a known fact that with silica gel based so called reversed phase supports the pH stability of the phase is restricted and is characteristically between pH 2-8. The phase bound to silica gel is less sensitive to acidic conditions and becomes sensitive to hydrolysis only below pH=2 [Sagliano Jr., N.; et al. Journal of Chromatography, 1988; 443:155-172], while above pH 8 hydroxide ions attack and dissolve silica gel, which results in a dramatic decline of efficiency [Kirkland, J.J.; et al. Journal of Chromatography, A 1995; 691:3-19]. Should in the process of surface modification remain free silanol groups, materials with basic groups may be bound to the surface, which entails a deterioration of selectivity [Nawrocki, J. Journal of Chromatography, A 1997; 779:29-71]. Modification by organosilanes the parts of the surface remaining free, s.c. end capping, resulted in supports of high stability, resistant up to pH=11.5 [Neue, U.D.; et al. American Laboratory 1999; 31 (22):36-39; Cheng, Y. F.; et al. LCGC 2000; 18(11):1162-1172].

Derived from the above knowledge our perception is that in contrast to EMAC supports known so far, in which it was the elaboration of ligand binding groups the main issue, the two advantages can be blended by the preparation of such IMAC supports in which, besides groups suitable to bind metals by chelation, the surface is also modified by inert groups unable to chelation. In this manner novel IMAC supports can be obtained in which the distance of chelating groups can be controlled and at the same time modification of an additional part of the surface by inert groups diminishes non-specific binding and increases the stability of the support.

With IMAC based protein binding and separations the use of Ni²⁺ or Co²⁺ metal ions is the most common [US Patent 4,877,830; Block, H.; et al. Methods in Enzymology, 2009; 463:439-473], while some methods mention and protect the use of other metal ions as well. Typical examples are US Patent 6,623,65, in which the metal of the metal ion are Ni, Hg, Ga, Cu, Ru, Co, Cd, Mg, Mn, Ti, In, Zn, Tc, Rh, Pd, Re, Fe, Au, Pb and Bi preferably Fe, Cu, Co, Au and Ni; US Patent 7,709,612 in which the metal of the metal ion is Ga and Fe; US Patent 7,005,071, in which the metal of the metal ion is Cu, Zn, Ni, Ca, Co, Mg, Fe, Au, Ga, Se. Certain methods specify in the description of the principle of the applicability of NTA based IMAC supports the ions Fe³⁺, Cu²⁺, Co²⁺, Ni²⁺, Zn²⁺, Ce³⁺, Mg²⁺, Ca²⁺, Ga³⁺, Cr³⁺, In³⁺, La³⁺, Lu³⁺, Sc³⁺, Ta³⁺, Yb³⁺, Th⁴⁺, U²⁺, Ag⁺, Au⁺ and Cu⁺, mentioning in the main claim metal ions in general (Mⁿ⁺, where n=1-6), while in further claims as metals only the use ofNi, Cu, Co, Fe, Zn and Ga are specified [US Patent 7,112,552; US Patent 7,304,015; US Patent 7,354,750].

In the course of the application of the most often used metal ions for binding and separation of proteins it is a serious disadvantage that, when used in larger quantities, the ions Ni²⁺ and Co²⁺ are toxic and carcinogenic. According to the standard EC No. 1272/2008 [EU-GHS/CLP] nickel acetate can be carcinogenic, it is harmful when ingested, on contact with the skin it may cause hypersensitivity, further it is of acute and chronic aquatic toxicity. Cobalt acetate has similar properties, which according to the guidelines of 67/548/EGK or 1999/45/EK can cause on inhaling cancer, can damage procreative capacity and fertility, may cause chronic health impairment, can result in sensibility on inhaling and on contact with the skin, as well it is extremely toxic for aquatic organisms and causing prolonged damage in an aquatic environment.

Lanthanide metals, earlier called "rare earth elements" [La, Ce, Pr, Nd, Pm, Sm, Eu, Gd, Tb, Dy, Ho, Er, Tm, Yb, Lu; which got their name from lanthanum (La) the first member of the series] are, in contrast to their name, not at all rare in the earth's crust; the general frequency of their occurrence is, with the exception of the radioactive promethium (Pr) produced artificially, commensurable with those of such elements of vital importance as iodine, selenium or cobalt [Palasz, A; Czekaj, P. Acta Biochimica Polonica, 2010; 47(4): 1107-1114]. Lanthanides are electropositive metals, in their stable compounds their oxidation number is generally +3, only Ce, Tb and Pm are forming stable compounds with an oxidation number of +4, while Sm, Eu and Yb with that of +2. In their compounds their coordination numbers are higher than 6, generally 8-9. The occurrence of lanthanides in the biosphere is rare and are unknown to be parts of biologically active compounds. Compared to other elements of non-vital importance toxicity of non-radioactive lanthanides is lower [Palasz, A; Czekaj, P. Acta Biochimica Polonica, 2010; 47(4): 1107-1114] and their ecological impact is similarly low [Tai, P.; et al., Chemosphere, 2010; 80(9): 1031-1035]. Lanthanides often exhibit special magnetic, catalytic or optical properties and therefore are widely utilized in industry and are relatively inexpensive.

The method which employs Tb-complexes of silica particles surface-modified with an EDTA-based chelating ligand for the luminescent detection of dipicolinic acid [Rieter, W, J.; et al, Journal of American Chemical Society, 2007; 129:9852-9853; Taylor K. M. L.; Lin, W. Journal of Materials Chemistry, 2009; 19:6418-6422] demonstrates, that lanthanide complexes of supports modified with appropriate ligands are suitable for binding molecules from a solution by complex formation along with optical change.

### DETAILED DESCRIPTION OF THE INVENTION

The objective of the invention is therefore a procedure for the preparation of IMAC supports of higher than usual stability and selectivity. Our perception is that by regulating the distance of ligands bound to the surface and suitable for binding of metal ions further by protecting an additional proportion of the solid surface by inert groups novel IMAC carriers can be gained the metal complexes of which, owing to the enhanced selectivity and stability of the novel IMAC supports, are especially suitable for the selective binding and separation of proteins having an adequate affinity to the metal ions.

We have found that the application of lanthanide ions - unexplored so far for IMAC based protein binding - offers special advantages, since the higher coordination ability of lanthanide ions permits the preparation of IMAC supports of higher than usual capacity and/or selectivity. A further advantageous feature of lanthanide based IMAC supports is that the toxicity of lanthanide ions, the appearance of which is unavoidable in the protein products are, compared to the usual metal ions, much less toxic, and also that based on the change of optical properties of the complexes protein binding and/or elution can be sensitively followed.

According to the above teaching the invention relates to a composition for binding and separating proteins which is the general formula (II) complexed with a lanthanide metal ion Mⁿ⁺ 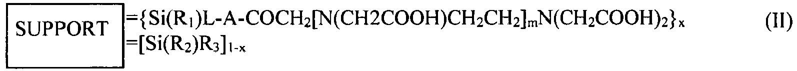 wherein
**R₁** and R₂ are C₁-C₈ straight chain or branched alkyl, or C₂-C₈ straight chain or branched alkenyl or alkynyl, C₆-C₈ aryl or aralkyl or cycloalkyl group; or alkoxy group,
**R₃** is R₂ or a non-reactive group which contains straight chain, branched or cyclic structures formed by 2 - 30 covalently connected carbon, oxygen, sulfur, fluorine, chlorine, bromine or iodine atoms,
**symbol** = represents one, two or three covalent single bonds between the Si atoms and the surface of the support, in case of one covalent bond, two of the R₁ - R₂ groups are attached to the given Si atom; in case of two covalent bonds, one of the R₁ - R₂ groups is attached to the given Si atom; in case of three covalent bonds, none of the groups R₁ - R₂ are attached to the given Si atom,
**A** is an atom or group of atoms containing a non-bonding pair of electrons, preferably NH, NR₂, S, O,
**L** is a straight, branched or cyclic structure connecting Si atoms and the A moiety comprising 2 - 22 carbon, sulfur or nitrogen atoms covalently connected to each other,
**x** is a number and 0 < x < 1,
m is an integer and m = 0, 1 or 2,
**M** is preferably low toxic, non-radioactive and M is selected from La, Ce, Nd, Pm, Sm, Eu, Gd, Dy, Ho, Er, Tm, Yb, Lu and
**n** is a number and n ≥ 1.

In addition to the above, the invention relates to the use of metal complexes of these compositions for the binding of such oligopeptides or proteins which contain affinity sequences of 5-12 amino acids long containing at least 3 histidines.

In a preferred use of metal complexes of compositions for the binding of such oligopeptides or proteins which contain affinity sequences of 5-12 amino acids long containing at least 3 histidines in such way that the change in the binding state of oligopeptides or proteins is followed by optical change.

In a preferred embodiment the composition wherein for the preparation of composition porous or fibrous solid carriers are used, typically porous support of 1 µm - 2 mm grain size and 4 - 200 nm pore diameter, or fibrous material with 100 - 2000 nm diameter with surfaces of silica-gel, aluminum oxide, titanium oxide, glass, cellulose or other material enabling the fabrication of structure with a general formula (II) by covalent bonds.

The new compositions and the procedure according to the invention is illustrated by the following examples, without limiting the scope of the invention to the examples given,

### Example 1

Silica gel (10 g, grain size: 40 - 63 µm, specific surface: 285 m²/g) was added to methanol (95%, 100 mL) containing 3-aminopropyltrimethoxysilane (2.0 mL; 11.5 mmol; 2.05 g) followed by shaking the suspension at room temperature for 168 hours. The product was filtered off and washed with methanol (3x100 mL), dried and stored at room temperature.
Elementary analysis: C: 1.78%, N: 1.12% H: 1.43%

### Example 2

The description according to Example 1 with the modification that the silane component is 3-(2-aminoethylamino)-propyldimethoxysilane (2.0 ml).
Elementary analysis: C: 4.02% N: 2.40% H: 1.83%

### Example 3

The description according to Example 1 with the modification that the silane component is 3-(2-aminoethylamino)-propylmethyldimethoxysilane (2.0 ml).
Elementary analysis: C: 3.22% N: 1.74% H: 1.71%

### Example 4

The description according to Example 1 with the modification that the silane component is a 1/3 (v/v) mixture of 3-aminopropyltrimethoxysilaneand phenyltrimethoxysilane (2.0 ml).
Elementary analysis: C: 6.37% N: 0.57% H: 1.48%

### Example 5

The description according to Example 1 with the modification that the silane component is a 1/3 (v/v) mixture of 3-(2-aminoethylamino)propyltrimethoxysilane and phenyltrimethoxysilane (2.0 ml). Elementary analysis: C: 6.26% N: 0.95% H: 1.59%

### Example 6

The description according to Example 1 with the modification that the silane component is a 1/3 (v/v) mixture of 3-(2-aminoethylamino)propylmethyldimethoxysilane / phenyltrimethoxysilane (2.0 ml). Elementary analysis: C: 5.66% N: 0.75% H: 1.48%

### Example 7

The description according to Example I with the modification that the silane component is a 1/3 (v/v) mixture of 3-aminopropyltrimethoxysilane and methyltrimethoxysilane (2.0 ml).
Elementary analysis: C: 1.56% N: 0.49% H: 1.37%

### Example 8

The description according to Example 1 with the modification that the silane component is a 1/3 (v/v) mixture of 3-(2-aminoethylamino)propyltrimethoxysilane and methyltrimethoxysilane (2.0 ml). Elementary analysis: C: 2.56% N: 1.03% H: 1.57%

### Example 9

The description according to Example 1 with the modification that the silane component is a 1/3 (v/v) mixture of 3-(2-aminoethylamino)propylmethyldimethoxysilane and methyltrimethoxysilane (2.0 ml). Elementary analysis: C: 2.51% N: 0.77% H: 1.44%

### Example 10

EDTA-dianhydride (300 mg, 1.17 mmol) was dissolved under inert conditions (argon atmosphere) in anhydrous *N,N*-dimethylformamide (DMF, 5.5 mL, 71.3 mmol, 5.2 g), then *N,N*-diisopropylethylamine (DEEA, 6 n/n%, based on DMF, 750 µl, 4.31 mmol, 557 mg) and the silica gel support (1.00 g) containing an amine functional group and prepared according to Example 1 was added. The mixture was stirred at 80 °C for 1 hour followed by the addition of distilled water (21 µl, 1.00 equivalent based on EDTA dianhydride). After continued stirring at 80 °C for 3 h the mixture was filtered followed by washing on the filter with DMF (3×5mL), distilled water (2×5 mL), saturated sodium hydrogen carbonate solution (2×5 mL) and anhydrous ethanol (2×5 mL). The product was dried in a vacuum desiccator over phosphorus pentoxide and stored.
Elementary analysis: C: 6.66% N: 2.08% H: 1.92%

### Example 11

The description according to Example 10 with the modification that the silica component is an amine group containing silica gel based support prepared according to Example 2 (1.00 g).
Elementary analysis: C: 10.06% N: 3.56% H: 2.47%

### Example 12

The description according to Example 10 with the modification that the silica component is an amine group containing silica gel based support prepared according to Example 3 (1.00 g).
Elementary analysis: C: 8.48% N: 2.74% H: 2.20%

### Example 13

The description according to Example 10 with the modification that the silica component is an amine group containing silica gel based support prepared according to Example 4 (1.00 g).
Elementary analysis: C: 8.59% N: 1.12% H: 1.70%

### Example 14

The description according to Example 10 with the modification that the silica component is an amine group containing silica gel based support prepared according to Example 5 (1.00 g).
Elementary analysis: C: 9.66% N: 1.47% H: 1.90%

### Example 15

The description according to Example 1.0 with the modification that the silica component is an amine group containing silica gel based support prepared according to Example 6 (1.00 g).
Elementary analysis: C: 8.10%N: 1.45% H: 1.80%

### Example 16

The description according to Example 10 with the modification that the silica component is an amine group containing silica gel based support prepared according to Example 7 (1.00 g).
Elementary analysis: C: 4.61%N: 1.14% H: 1.6.8%

### Example 17

The description according to Example 10 with the modification that the silica component is an amine group containing silica gel based support prepared according to Example 8 (1.00 g).
Elementary analysis: C: 4.61% N: 1.14% H: 1.68%

### Example 18

The description according to Example 10 with the modification that the silica component is an amine group containing silica gel based support prepared according to Example 9 (1.00 g).
Elementary analysis: C: 6.32% N: 1.72% H: 1.91%

The following examples 19-37 are reference examples not falling within the scope of the claims.

### Example 19

The silica gel based support coupled with EDTA anhydride prepared as presented in Example 10 (1.00 g) was suspended in a nickel chloride solution (NiCl₂; 100 mM; 16 mL) then, after shaking for 5 min it was filtered off. The filtered silica gel based IMAC support was washed with HEPES buffer (2-[4-(2-hydroxyethyl)piperazin-1-yl]ethanesulfonic acid; 20 mM; pH=7.5; 16 mL), distilled water (20 mL), imidazole solution (500 mM; pH=7.5; 9 ml), than with water (20 mL). The Ni containing silica gel based support prepared was stored at room temperature under 20% ethanol.
Flame-AAS: Ni: 2.00%

### Example 20

The description according to Example 19 with the modification that the IMAC support was a silica gel based support coupled with EDTA anhydride prepared in a way presented in Example 11 (1.00 g). Flame-AAS: Ni: 2.61%

### Example 21

The description according to Example 19 with the modification that the IMAC support was a silica gel based support coupled with EDTA anhydride prepared in a way presented in Example 12 (1.00 g). Flame-AAS: Ni: 2.71%

### Example 22

The description according to Example 19 with the modification that the IMAC support was a silica gel based support coupled with EDTA anhydride prepared in a way presented in Example 13 (1.00 g). Flame-AAS: Ni: 1.20%

### Example 23

The description according to Example 19 with the modification that the IMAC support was a silica gel based support coupled with EDTA anhydride prepared in a way presented in Example 14 (1.00 g). Flame-AAS: Ni: 1.81%

### Example 24

The description according to Example 19 with the modification that the MAC support was a silica gel based support coupled with EDTA anhydride prepared in a way presented in Example 15 (1.00 g).
Flame-AAS: Ni; 1.4.9%

### Example 25

The description according to Example 19 with the modification that the LMAC support was a silica gel based support coupled with EDTA anhydride prepared in a way presented in Example 16 (1.00 g). Flame-AAS: Ni: 1.26%

### Example 26

The description according to Example 19 with the modification that the MAC support was a silica gel based support coupled with EDTA anhydride prepared in a way presented in Example 17 (1.00 g). Flame-AAS: Ni: 1.68%

### Example 27

The description according to Example 19 with the modification that the MAC support was a silica gel based support coupled with EDTA anhydride prepared in a way presented in Example 18 (1.00 g). Flame-AAS: Ni: 1.79%

### Example 28

*Escherichia coli* Rosetta (DE3) pLys (NOVAGEN) cells were transformed with pET-19b plasmids containing a *Drosophila virilis* dUTPase enzyme coding gene sequence (Dv dUTPase, 530 amino acids, 57.9 kDa, pI 5.94) [The Drosophila Consortium, Nature, 2007; 450(7167):203-218] and ampicillin resistance. When the enzyme is produced with this vector, the A-terminal of the protein contains a His₆ tag. The transformed cells were selected with the aid of LB agarose plates (10 g Tripton, 5 g yeast extract, 10 g NaCl, 18.75 g microbiologic agar /1 L, pH 7.5) containing 0.1 mg/mL ampicillin, With the plasmid containing cells we prepared cultures of 5 mL volume (200 rpm, 37 °C, 16 h), and then this inoculum was transferred into 500 mL of culture medium. The cells grown at 37 °C and shaken at 200 rpm were induced after attaining an optical density of 0.5 with 0.6 mM IPTG(isopropyl-β-D-1-thiogalactopyranoside). After adding the inducing agent protein production was carried out by shaking at 25 °C (4 hours, 200 rpm). Thereafter the cells were centrifuged (3600 rpm, 20 min, 4 °C) and the cell pellets suspended in 50 mL of a lysis buffer at 4 °C (50 mM TRIS-HC1, pH 7.5, 300 mM NaCl, 0.5 mM EDTA, 5 mM β-mercaptoethanol, 1 mM PMSF [phenylmethanesulfonylfluoride], 5 mM BA (benzyladenine), 0.1 mg/mL lysozyme, 0.1 mg/mL DNAse, 0.01 mg/mL RNAse A). Thereafter the suspended cells were disrupted by sonication (Bandelin Sonopuls HD 2070 apparatus, 10x30 sec, amplitude 40%, 6 cycles/min) with ice cooling; Separation of the cell debris from the supernatant intended for the testing of the affinity material was carried out by centrifuging (5000 rpm, 20 min, 4 °C).

### Example 29

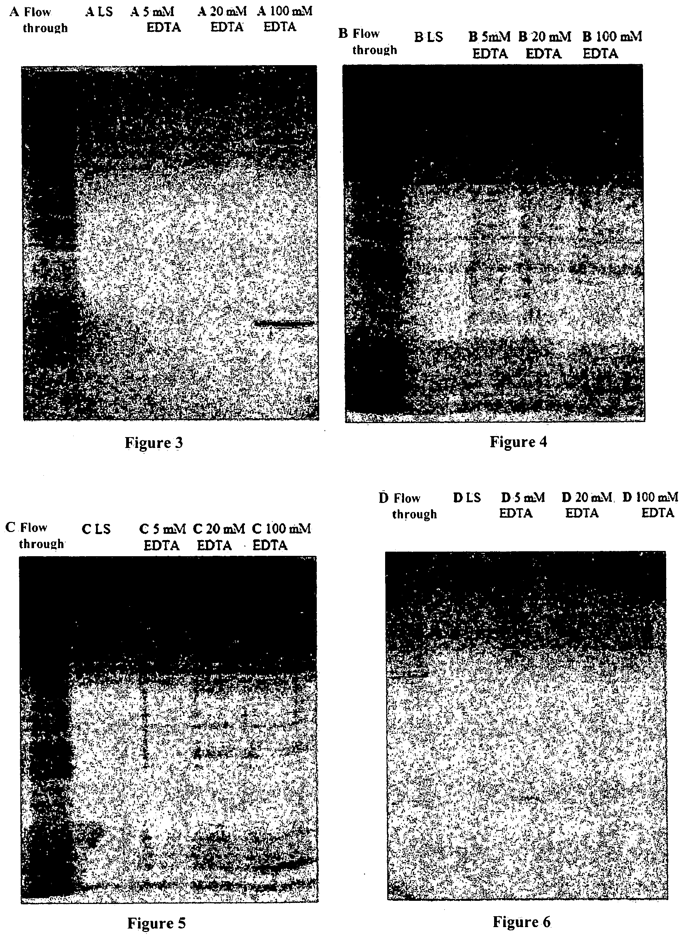

The Ni-containing affinity material (20.0 mg) prepared in a manner presented in Example 19 was suspended in distilled water (1 mL) and suspended again after standing for 15 min. The sample was centrifuged (5000 rpm, 25 °C, 10 min) and the water decanted. To the wet affinity material, the supernatant of Dv dUTPase prepared as shown in Example 28 (100 µL, 7.8 mg total protein/mL supernatant) was added, the sample suspended, centrifuged (5000 rpm, 25 °C, 10 min) and the supernatant decanted (Figure 3, "A Flow through"). The sample was washed with Low Salt buffer (LS, 2×1 mL, 50 mM HEPES [2-[4-(2-hydroxyethyl)piperazin-1-yl]ethanesulfonic acid], 30 mM KCl, pH 1-7.5), centrifuged (5000 rpm, 25 °C, 10 min) and the supernatant decanted (Figure 3, "A LS"). Thereafter the sample was washed with EDTA Solutions of increasing concentration (100 µL, 5 mM, pH=8.0; 100 µL, 20 mM, pH=8.0; 100 µL, 100 mM, pH=8.0). After each washing the suspended sample was centrifuged (5000 rpm, 25 °C, 10 min) and decanted (Figure 3, "A 5 mM EDTA", "A 20 mM EDTA", "A 100 mM EDTA"). Finally, the IMAC support was washed with distilled water (3×1 mL) and stored under 20% ethanol. To the samples from the centrifuged supernatants (40 µL) dye solution (10 µL; 250 mM Tris-HCl pH 6.8; 10% SDS [sodium dodecylsulfate]; 10% DTT [dithiothreitol]; 50% glycerol; 0.05% bromophenol-blue dye) was added and then from this mixture followed by heat treatment at 95 °C (5 min) 12.5 µL was pipette into the sample holding wells of the electrophoresis gel. Gel electrophoresis was carried out on a 12% acrylamide gel (electrophoresis buffer: 3.03 g TRIS, 14.4 g glycine, 1.0 g SDS, Bio Rad Mini-PROTEAN® Tetra cell running tank, PowerPac Universal power supply, voltage 200 V).

### Example 30

The procedure presented in Example 29 modified in a way that the Ni-containing affinity-material was prepared as presented in Example 20.

(Figure 4; "B Flow through"; "B LS"; "B 5 mM EDTA"; "B 20 mM EDTA"; "B 100 mM EDTA").

### Example,31

The procedure presented in Example 29 modified in a way that the Ni-containing affinity material was prepared as presented in Example 21.
(Figure 5; "C Flow through"; "C LS"; "C 5 mM EDTA"; "C 20 mM EDTA"; "C 100 mM EDTA").

### Example 32

The procedure presented in Example 29 modified in a way that the Ni-containing affinity material was prepared as presented in Example 22.
(Figure 6; "D Flow through"; "D LS"; "D 5 mM EDTA"; "D 20 mM EDTA"; "D 100 mM EDTA").

### Example 33

The procedure presented in Example 29 modified in a way that the Ni-containing affinity material was prepared as presented in Example 23.

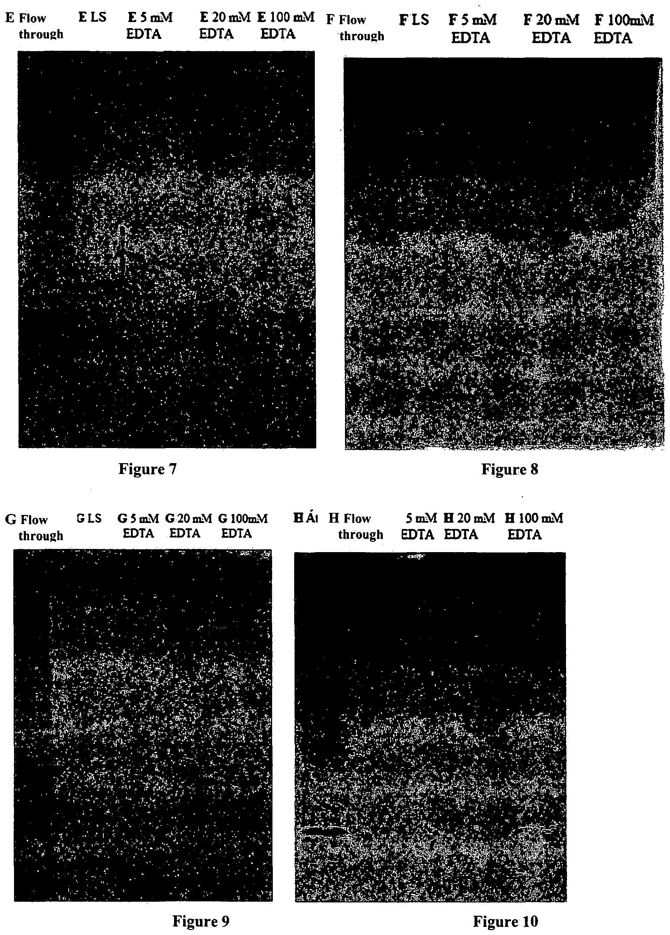

(Figure 7; "E Flow through"; "E LS"; "E 5 mM EDTA"; "E 20 mM EDTA"; "E 100 mM EDTA").

### Example 34

The procedure presented in Example 29 modified in a way that the Ni-containing affinity material was prepared as presented in Example 24.
(Figure 8; "F Flow through"; "F LS"; "F 5 mM EDTA"; "F 20 mM EDTA"; "F 100 mM EDTA").

### Example 35

The procedure presented in Example 29 modified in a way that the Ni-containing affinity material was prepared as presented in Example 20.
(Figure 9; "G Flow through"; "G LS"; "G 5 mM EDTA"; "G 20 mM EDTA"; "G 100 mM EDTA").

### Example 36

The procedure presented in Example 29 modified in a way that the Ni-containing affinity material was prepared as presented in Example 26.
(Figure 10; "H Flow through"; "H LS"; "H 5 mM EDTA"; "H 20 mM EDTA"; "H 100 mM EDTA").

### Example 37

The procedure presented in Example 29 modified in a way that the Ni-containing affinity material was prepared as presented in Example 27.
(Figure 11; "I Flow through"; "I LS"; "I 5 mM EDTA"; "I 20 mM EDTA"; "I 100 mM EDTA").

### Example 38

Description according to Example 19 modified, in a way that the affinity material was a silica gel based support (1.00 g) coupled with EDTA as chelating agent prepared in a way presented in Example 10 and into which the lanthanide metal ion was introduced with lanthanum nitrate (La(NO₃)₃; 100 mM; 16 mL).

### Example 39

Description according to Example 19 modified in a way that the affinity material was a silica gel based support (1.00 g) coupled with EDTA as chelating agent prepared in a way presented in Example 12 and into which the lanthanide metal ion was introduced with lanthanum nitrate (La(NO₃)₃; 100 mM; 16 mL).

### Example 40

Description according to Example 19 modified in, a way that the affinity material was a silica gel 10 based support (1.00 g) coupled with EDTA as chelating agent prepared in a way presented in Example 16 and into which the lanthanide metal ion was introduced with lanthanum nitrate (La(NO₃)₃; 100 mM; 16 mL).

### Example 41

Description according to Example 19 modified in a way that the affinity material was a silica gel based support (1,00 g) coupled with EDTA as chelating agent prepared in a way presented in Example 18 and into which the lanthanide metal ion was introduced with lanthanum nitrate (La(NO₃)₃; 100 mM; 16 mL).

### Example 42

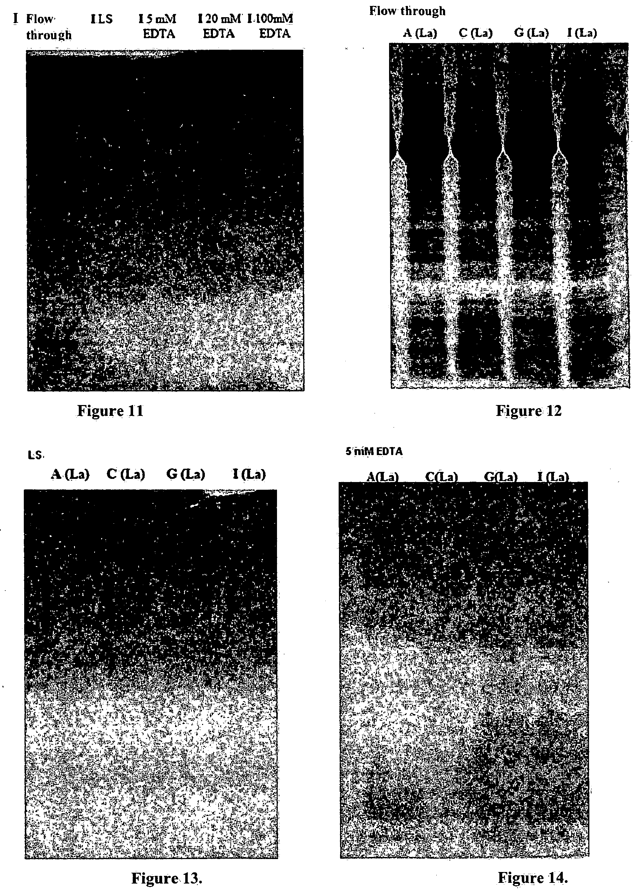

The procedure presented in Example 29 modified in a way that the La³⁺containing affinity material was prepared according to the method presented in Example 38 (Figures 12-16, "A").

### Example 43

The procedure presented in Example 29 modified in a way that the La³⁺ containing affinity material was prepared according to the method presented in Example 39 (Figures 12-16, "C").

### Example 44

The procedure presented in Example 29 modified in a way that the La³⁺containing affinity material was prepared according to the method presented in Example 40 (Figures 12-16, "G").

### Example 45

The procedure presented in Example 29 modified in a way that the La³⁺ containing affinity material was prepared according to the method presented in Example 41 (Figures 12-16, "I").

### Example 46

From a silica sol [a mixture in a molar proportion of 1 : 2 : 2 : 0.01 of tetraethoxysilane (TEOS), ethanol, distilled water and HCl treated at 80 °C for 30 min] utilizing the disclosed method of electrospinning in the known way [Choi, S. S.; et al. Journal of Materials Science Letters, 2003; 22:891-893] employing 12 kV voltage, a tissue of 0.5 - 1 mm thickness consisting of silica nano-fibers of 500 - 1200 nm diameter was produced.

### Example 47

The description according to Example 4 modified in a way that the solid support was 100 mg of silica nano-fiber tissue (accordingly, compared with Example 4, one hundredths of solvent and silane reagent was used).

### Example 48

The description according to Example 5 modified in a way that the solid support was 100 mg of silica nano-fiber tissue (accordingly, compared with Example 5, one hundredths of solvent and silane reagent was used).

### Example 49

The description according to Example 6 modified in a way that the solid support was 100 mg of silica nano-fiber tissue (accordingly, compared with Example 6, one hundredths of solvent and silane reagent was used).

### Example 50

The description according to Example 7 modified in a way that the solid support was 100 mg of silica nano-fiber tissue (accordingly, compared with Example 7, one hundredths of solvent and silane reagent was used).

### Example 51

The description according to Example 8 modified in a way that the solid support was 100 mg of silica nano-fiber tissue (accordingly, compared with Example 8, one hundredths of solvent and silane reagent was used).

### Example 52

The description according to Example 9 modified in a way that the solid support was 100 mg of silica nano-fiber tissue (accordingly, compared with Example 9, one hundredths of solvent and silane reagent was used).

### Example 53

The description according to Example 10 modified in a way that the solid component was a silica nano-fiber based support (100 mg) prepared according to the method presented in Example 47 containing an amino functional group (accordingly, compared with Example 10, applying one tenth of solvent and EDTA dianhydride).

### Example 54

The description according to Example 10 modified in a way that the solid component was a silica nano-fiber based support (100 mg) prepared according to the method presented in Example 48 containing an amino functional group (accordingly, compared with Example 10, applying one tenth of solvent and EDTA dianhydride).

### Example 55

The description according to Example 10 modified in a way that the solid component was a silica nano-fiber based support (100 mg) prepared according to the method presented in Example 49 containing an amino functional group (accordingly, compared with Example 10, applying one tenth of solvent and EDTA dianhydride).

### Example 56

The description according to Example 10 modified in a way that the solid component was a silica nano-fiber based support (100 mg) prepared according to the method presented in Example 50 containing an amino functional group (accordingly, compared with Example 10, applying one tenth of solvent and EDTA dianhydride).

### Example 57

The description according to Example 10 modified in a way that the solid component was a silica nano-fiber based support (100 mg) prepared according to the method presented in Example 51 containing an amino functional group (accordingly, compared with Example 10, applying one tenth of solvent and EDTA dianhydride).

### Example 58

The description according to Example 10 modified in a way that the solid component was a silica nano-fiber based support (100 mg) prepared according to the method presented in Example 52 containing an amino functional group (accordingly, compared with Example 10, applying one tenth of solvent and EDTA dianhydride).

### Example 59

The description according to Example 10 modified in a way that the solid component was a silica nano-fiber based support (100 mg) prepared according to the method presented in Example 53 containing an amino functional group (accordingly, compared with Example 10, applying one tenth of solvent and EDTA dianhydride).

### Example 60

The description according to Example 19 modified in a way that the solid support was an EDTA functionalized silica nano-fiber based support (100 mg) prepared in a way presented in Example 54 (accordingly, compared with Example 19, one tenth of washing solution was applied).

### Example 61

The description according to Example 19 modified in a way that the solid support was an EDTA functionalized silica nano-fiber based support (100 mg) prepared in a way presented in Example 55 (accordingly, compared with Example 19, one tenth of washing solution was applied).

### Example 62

The description according to Example 19 modified in a way that the solid support was an EDTA functionalized silica nano-fiber based support (100 mg) prepared in a way presented in Example 56 (accordingly, compared with Example 19, one tenth of washing solution was applied).

### Example 63

The description according to Example 19 modified in a way that the solid support was an EDTA functionalized silica nano-fiber based support (100 mg) prepared in a way presented in Example 57 (accordingly, compared with Example 19, one tenth of washing solution was applied).

### Example 64

The description according to Example 19 modified in a way that the solid support was an EDTA functionalized silica nano-fiber based support (100 mg) prepared in a way presented m Example 58 (accordingly, compared with Example 19, one tenth of washing solution was applied).

### Example 65

The description according to Example 38 modified in a way that the solid support was an EDTA functionalized silica nano-fiber based support (100 mg) prepared in a way presented in Example 53 (accordingly, compared with Example 38, one tenth of washing solution was applied).

### Example 66

The description according to Example 38 modified in a way that the solid support was an EDTA functionalized silica nano-fiber based support (100 mg) prepared in a way presented in Example 54 (accordingly, compared with Example 38, one tenth of washing solution was applied).

### Example 67

The description according to Example 38 modified in a way that the solid support was an EDTA functionalized silica nano-fiber based support (100 mg) prepared in a way presented in Example 55 (accordingly, compared with Example 38, one tenth of washing solution was applied).

### Example 68

The description according to Example 38 modified in a way that the solid support was an EDTA functionalized silica nano-fiber based support (100 mg) prepared in a way presented in Example 56 (accordingly, compared with Example 38, one tenth of washing solution was applied).

### Example 69

The description according to Example 38 modified in a way that the solid support was an EDTA functionalized silica nano-fiber based support (100 mg) prepared in a way presented in Example 57 (accordingly, compared with Example 38, one tenth of washing solution was applied).

### Example 70

The description according to Example 38 modified in a way that the solid support was an EDTA functionalized silica nano-fiber based support (100 mg) prepared in a way presented in Example 58 (accordingly, compared with Example 38, one tenth of washing solution was applied).

### Example 71

The description according to Example 1 with the modification that the silane component is a 1/2.6 (y/v) mixture of 3-(2-aminoethylamino)propyltrimethoxysilane and methyltrimethoxysilane (2.0 ml).
Elementary analysis: C: 2.76% N: 0.85% H: 1.58%

### Example 72

The description according to Example 10 with the modification that the silica component is an amine group containing silica gel based support prepared according to Example 71 (1.00 g).
Elementary analysis: C: 5,09% N: 1.26% H: 1.85%

### Reference example 73

The description according to Example 19 with the modification that the IMAC support was a silica gel based support coupled with EDTA anhydride prepared in a way presented in Example 72 (1.00 g).
Flame-AAS: Ni: 2.21%

### Reference example 74

The description according to Example 19 modified in a way that the affinity material was a silica gel based support (1.00 g) coupled with EDTA as chelating agent prepared in a way presented in Example 72 and into which the metal ion was introduced with cobalt chloride (CoCl₂; 100 mM; 16 mL).

### Example 75

The description according to Example 19 modified in a way that the affinity material was a silica gel based support (1.00 g) coupled with EDTA as chelating agent prepared in a way presented in Example 72 and into which the lanthanide metal ion was introduced with lanthanum nitrate ((La(NO₃)₃; 100 mM; 16 mL).

### Example 76

The description according to Example 19 modified in a way that the affinity material was a silica gel based support (1.00 g) coupled with EDTA as chelating agent prepared in a way presented in Example 72 and into which the lanthanide metal ion was introduced with europium acetate (Eu(OAc)₃; 100 mM; 16 mL).

### Reference example 77

Description according to Example 19 modified in a way that the affinity material was a silica gel based support (1.00 g) coupled with EDTA as chelating agent prepared in a way presented in Example 72 and into which the lanthanide metal ion was introduced with terbium acetate (Tb(OAc)₃; 100 mM; 16 mL).

### Example 78

*Escherichia coli* Rosetta (DE3) pLys (NOVAGEN) cells were transformed with pET-19b plasmids containing a *Homo sapiens* dUTPase enzyme coding gene sequence (*Hs* dUTPase, 184 amino acids, 19.9 kDa, pI 7.13) [Miyahara, S.; et al. Journal of Medicinal Chemistry, 2012; 55(11):5483-5496] and ampicillin resistance. When the enzyme is produced with this vector, the *N*-terminal of the protein contains a His₆ tag. The transformed cells were selected with the aid of LB agarose plates (10 g Tripton, 5 g yeast extract, 10 g NaCl, 18.75 g microbiologic agar/1 L, pH 7.5) containing 0.1 mg/mL ampicillin. With the plasmid containing cells we prepared cultures of 5 mL volume (200 rpm, 37 °C, ca. 12 h), and then this inoculum was transferred into 500 mL of culture medium. The cells grown at 37 °C and shaken at 200 rpm were induced after attaining an optical density of 0.5 with 0.1 mM IPTG (isopropyl-β-D-1-thiogalactopyranoside) at 30 °C. After adding the inducing agent protein production was carried out by shaking at 28 °C (6 h, 200 rpm). Thereafter the cells were centrifuged (3600 rpm, 20 min 4 °C) and the cell pellets suspended in 50 mL of a lysis buffer at 4 °C (50 mM TRIS-HCl, pH 7.5, 300 mM NaCl, 0.5 mM EDTA, 5 mM p.-mercaptoethanol, 1 mM PMSF [phenylmethanesulfonylfluoride], 5 mM BA (benzyladenine), 0.1 mg/mL lysozyme, 0.1 mg/mL DNAse, 0.01 mg/mL RNAse A). Thereafter the suspended cells were disrupted by sonication (Bandelin Sonopuls HD 2070 apparatus, 10X30 sec, amplitude 40%, 6 cycles/min) with ice cooling. Separation of the cell debris from the supernatant intended for the testing of the affinity material was carried out by centrifuging (5000 rpm, 20 min, 4 °C).

### Reference example 79

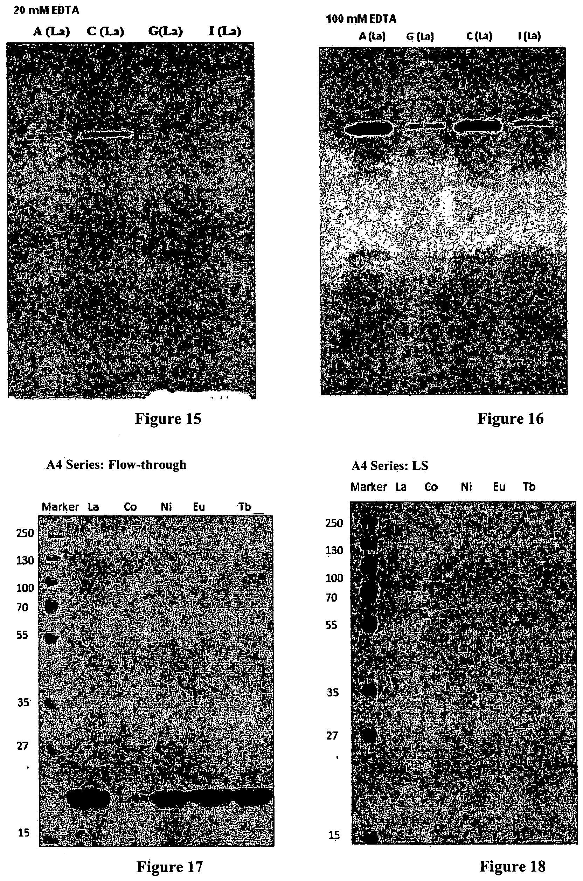

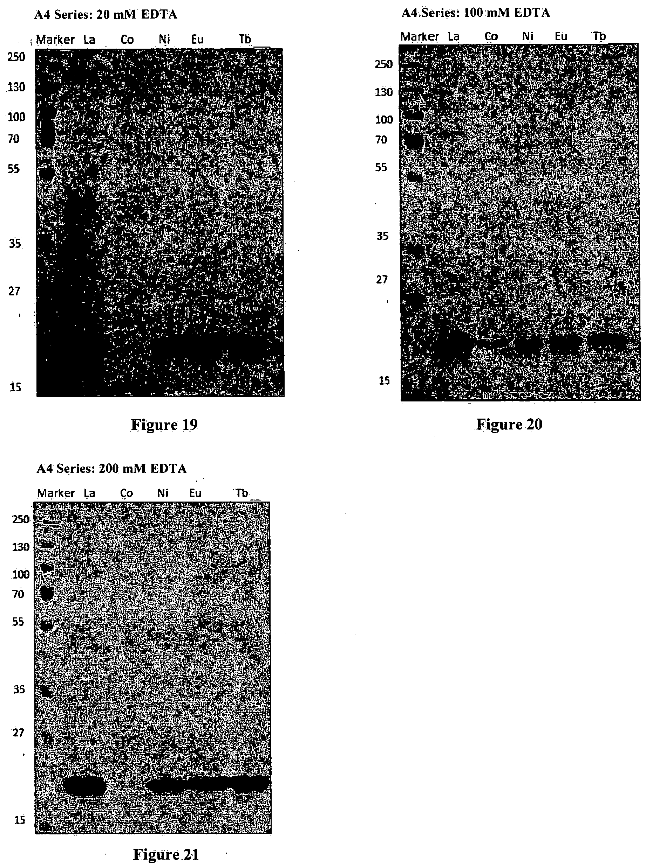

The Ni²⁺-containing affinity material (20.0 mg) prepared in a manner presented in Example 73 was suspended in distilled water (1 mL) and suspended again after standing for 15 min. The sample was centrifuged (5000 rpm, 25 °C, 10 min) and the water decanted. To the wet affinity material the supernatant of *Hs* dUTPase prepared as shown in Example 78 (100 µL, 7.8 mg total protein/mL supernatant) was added, the sample shake for 2 h, centrifuged (5000 rpm, 25 °C, 10 min) and the supernatant decanted (Figure 17, "A4 Series: Flow through", Lane Ni). The sample was washed with Low Salt buffer (LS, 2x1 mL, 50 mM HEPES [2-[4-(2-hydroxyethyl)piperazine-1-yl]ethanesulfonic acid], 30 mM KC1, pH=7.5), centrifuged (5000 rpm, 25 °C, 10 minutes) and the supernatant decanted (Figure 18, "A4 Series: LS", Lane "Ni"). Thereafter the sample was washed with EDTA Solutions of increasing concentration (100 µl, 5 mM, pH=8.0; 100 µl, 20 mM, pH=8.0; 100 µl, 100 mM, pH=8.0; 100 µl, 200 mM, pH=8.0). After each washing the suspended sample was centrifuged (5000 rpm, 25 °C, 10 min) and decanted (Figure 19, "A4 Series: 20 mM EDTA", Lane "Ni"; Figure 20, "A4 Series: 100 mM EDTA", Lane "Ni"; Figure 21, "A4 Series: 200 mM EDTA", Lane "Ni"). Finally, the IMAC support was washed with distilled water (3x1 mL) and stored under 20% ethanol. To the samples from the centrifuged supernatants (40 µl) dye solution (10 µl; 250 mM Tris-HCl pH 6.8; 10% SDS [sodium dodecylsulfate]; 10% DTT [dithiothreitol]; 50% glycerol; 0.05% bromophenol-blue dye) was added and then from this mixture followed by heat treatment at 95 °C (5 min) 12.5 µl was pipetted into the sample holding wells of the electrophoresis gel. Gel electrophoresis was carried out on a 12% acrylamide gel (electrophoresis buffer: 3.03 g TRIS, 14.4 g glycine, 1.0 g SDS, Bio Rad Mini-PROTEAN® Tetra cell running tank, Power Pac Universal power supply, voltage 200 V).

### Reference example 80

The procedure presented in Example 79 modified in a way that the Co²⁺ containing affinity material was prepared according to the method presented in Example 74 (Figures 17-21, Lanes "Co").

### Example 81

The procedure presented in Example 79 modified in a way that the La³⁺ containing affinity material was prepared according to the method presented in Example 75 (Figures 17-21, Lanes "La").

### Example 82

The procedure presented in Example 79 modified in a way that the Eu³⁺ containing affinity material was prepared according to the method presented in Example 76 (Figures 17-21, Lanes "Eu").

### Reference example 83

The procedure presented in Example 79 modified in a way that the Tb³⁺ containing affinity material was prepared according to the method presented in Example 77 (Figures 17-21, Lanes "Tb"

## Claims

1. A composition for binding and separating proteins **characterized by** the general formula (II) complexed with a lanthanide metal ion Mⁿ⁺ 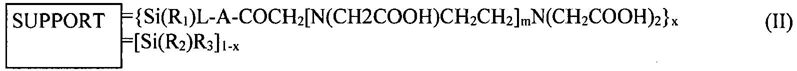 wherein
**R₁** and **R₂** are C₁-C₈ straight chain or branched alkyl, or C₂-C₈ straight chain or branched alkenyl or alkynyl, C₆-C₈ aryl or aralkyl or cycloalkyl group; or alkoxy group,
**R₃** is R₂ or a non-reactive group which contains straight chain, branched or cyclic structures formed by 2 - 30 covalently connected carbon, oxygen, sulfur, fluorine, chlorine, bromine or iodine atoms,
**symbol** = represents one, two or three covalent single bonds between the Si atoms and the surface of the support, in case of one covalent bond, two of the R₁ - R₂ groups are attached to the given Si atom; in case of two covalent bonds, one of the R₁ - R₂ groups is attached to the given Si atom; in case of three covalent bonds, no R₁ - R₂ groups are attached to the given Si atom,
**A** is an atom or group of atoms containing a non-bonding pair of electrons, preferably NH, NR₂, S, O,
**L** is a straight, branched or cyclic structure connecting Si atoms and the A moiety comprising 2 - 22 carbon, sulfur or nitrogen atoms covalently connected to each other,
**x** is a number and 0 < x < 1
**m** is an integer and m = 0, 1 or 2,
**M** is a lanthanide metal chosen from La, Ce, Nd, Pm, Sm, Eu, Gd, Dy, Ho, Er, Tm, Yb, Lu atoms and
**n** is a number and n ≥ 1.

2. Use of lanthanide metal complexes of compositions as claimed in Claim 1 for the binding of such oligopeptides or proteins which contain affinity sequences of 5-12 amino acids long containing at least 3 histidines.

3. Use of lanthanide metal complexes of compositions as claimed in Claim 1 for the binding of such oligopeptides or proteins which contain affinity sequences of 5-12 amino acids long containing at least 3 histidines in such way that the change in the binding state of oligopeptides or proteins is followed by optical change.

4. A composition as claimed in Claim 1 wherein for the preparation of composition porous or fibrous solid carriers arc used, typically porous support of 1 µm - 2 mm grain size and 4 - 200 nm pore diameter, or fibrous material with 100 - 2000 nm diameter with surfaces of silica-gel, aluminum oxide, titanium oxide, glass, cellulose or other material enabling the fabrication of structure with a general formula (II) by covalent bonds.

## Patentansprüche

1. Mit Lanthanoidmetall-Ionen Mⁿ⁺ gebildete, zur Bindung und Trennung von Proteinen geeignete Komplexe von Trägern der allgemeinen Struktur (II), 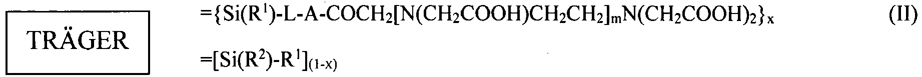 worin
**R¹** und **R²** für eine gerad- oder verzweigtkettige (C₁-C₈)-Alkyl-; eine gerad- oder verzweigtkettige (C₂-C₈)-Alkenyl- oder Alkinyl-; oder eine (C₆-C₈)-Aryl-, Aralkyl- oder Cycloalkylgruppe; oder eine Alkoxygruppe stehen,
**R₃** die Bedeutung von R² oder von einer nichtreaktiven Gruppe hat, die eine gerad-, verzweigtkettige oder cyclische Struktur enthält, die aus 2 - 30, untereinander in kovalenter Beziehung stehenden Kohlenstoff-, Sauerstoff-, Schwefel-, Fluor-, Chlor-, Brom- oder Iodatomen gebildet wird,
das **Symbol** = die Bedeutung von
ein, zwei oder drei kovalenten Einfachbindungen zwischen dem Si-Atom und der Oberfläche des Trägers hat; im Falle von einer kovalenten Bindung sind von den Gruppen R¹ und R² zwei an das betreffende Si-Atom gebunden; im Falle von zwei kovalenten Bindungen ist von den Gruppen R¹ und R² eine an das betreffende Si-Atom gebunden; im Falle von drei kovalenten Bindungen ist von den Gruppen R¹ und R² keine an das betreffende Si-Atom gebunden,
**A** ein Atom oder eine Atomgruppe ist, das/die ein nichtbindendes Elektronenpaar beinhaltet, vorzugsweise NH, NR₂, S, O,
**L** eine gerad-, verzweigtkettige oder cyclische Strukturen beinhaltende Einheit ist, die das Si-Atom mit der Einheit A verbindet und 2-22 Kohlenstoff-, Schwefel- beziehungsweise Stickstoffatome beinhaltet,
**x** eine Zahl ist und 0 >x> 1,
**m** eine ganze Zahl ist und m = 0, 1 oder 2,
**M** ein Lanthanoidmetall unter den Atomen La, Ce, Nd, Pm, Sm, Eu, Gd, Dy, Ho, Er, Tm, Yb, Lu ist und
**n** eine ganze Zahl bedeutet und n ≥ 1 ist.

2. Die Verwendung von mit Lanthanoidmetall-Ionen komplexierten Präparaten nach Anspruch 1 zur Bindung von Oligopeptiden oder Proteinen, die Affinitätsabschnitte mit einer Länge von 5-12 Aminosäuren - darunter mindestens 3 Histidingruppen - enthalten.

3. Die Verwendung von mit Lanthanoidmetall-Ionen komplexierten Präparaten nach Anspruch 1 zur Bindung von Oligopeptiden oder Proteinen, die Affinitätsabschnitte mit einer Länge von 5-12 Aminosäuren - darunter mindestens 3 Histidingruppen - enthalten, wobei die Bindungszustandsänderung der Oligopeptide oder der Proteine auf optischem Wege verfolgt wird.

4. Ein Präparat nach Anspruch 1 **dadurch gekennzeichnet, dass** man für deren Herstellung eine poröse oder faserartige Trägersubstanz verwendet, vorzugsweise eine poröse Trägersubstanz, deren Korngröße 1 µm - 2 mm und Porengröße 4 - 200 nm beträgt, oder eine faserartige Trägersubstanz, deren Durchmesser 100 - 2000 nm beträgt und deren Oberfläche Kieselsäuregel, Aluminiumoxid, Titandioxid, Glas, Zellulose oder ein anderes Material ist, das die mittels kovalenter Bindungen erfolgende Bildung der mit der allgemeinen Formel (II) gekennzeichneten Struktur ermöglicht.

## Revendications

1. Complexes des supports caractérisables par la structure générale (II) formés avec des ions métalliques lanthanides Mⁿ⁺, 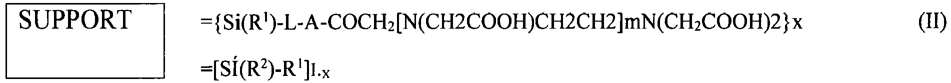 où
**R¹** et **R²** sont un groupe alkyl droit ou ramifié ayant une structure de C1 à C₈ atomes de carbone, ou un groupe alkynyl ou bien alkenyl droit ou ramifié ou encore un groupe aryle, arakyle ou cycloalkyle ayant de C₂ à C₈ atomes ; ou un groupe alkoxy,
**R³** signifie R², ou un groupe non réactif lequel comprend une structure droite, ramifiée ou cyclique composée de 2 à 30 atomes de carbone, oxygène, soufre, fluor, chlore, brome, ou iode liés par des liaisons covalentes,
a = signification du **symbole**
Une, deux ou trois liaisons covalentes simples entre l'atome Si et la surface du support ; dans le cas d'une liaison covalente, deux parmi les groupes R¹ et R² se lient à l'atome Si donné; dans le cas de deux liaisons covalentes un parmi les groupes R¹ et R² se lie à l'atome Si donné ; dans le cas de trois liaisons covalentes aucun parmi les groupes R¹ et R² ne se lie à l'atome Si donné,
**A** est un atome ou un groupe d'atomes qui comporte un doublet non liant, de préférence NH, NR₂, S, O,
**L** est une unité comportant des structures droites, ramifiées ou cycliques qui lie l'atome Si et l'unité A et contient de 2 à 22 atomes de carbone, de soufre ou d'azote,
**x** est un nombre qui répond à l'intervalle de valeur suivant 0 >x> 1,
**m** est un nombre entier et m = 0, 1 ou 2,
**M** est un lanthanide, parmi lesquels on trouve les atomes suivants La, Ce, Nd, Pm, Sm, Eu, Gd, Dy, Ho, Er, Tm, Yb, Lu et
**n** signifie un nombre entier supérieur ou égal à 1. n ≥ 1.

2. Utilisation des préparations formant un complexe avec des ions métalliques lanthanides conformes au périmètre du brevet numéro 1 pour l'adsorption des oligopeptides ou des protéines contenant des sections de longueur de 5 à 12 acides aminés intégrant au moins 3 histidines séparées par chromatographie d'affinité.

3. Utilisation des préparations formant un complexe avec des ions métalliques lanthanides conformes au périmètre du brevet numéro 1 pour l'adsorption des oligopeptides ou des protéines contenant des sections de longueur de 5 à 12 acides aminés intégrant au moins 3 histidines séparées par chromatographie d'affinité et ce de telle manière que l'évolution de l'état d'adsorption des oligopeptides ou des protéines est suivi par la voie optique.

4. La préparation conforme au périmètre du brevet numéro 1 avec la précision que un support poreux ou fibreux est utilisé pour leur réalisation, de préférence un support poreux dont la granulométrie se situe entre 1 pm et 2 mm et la taille des pores évaluée entre 4 et 200 nm ou un support fibreux dont le diamètre se situe entre 100 et 2000 nm avec à sa surface un gel de silice, ou d' oxyde d'aluminium, d'oxyde de titane, de verre, de cellulose ou d'autre matière permettant la formation de la structure **caractérisée par** la formule générale (II) avec des liaisons covalentes.
